Europäisches Patentamt

European Patent Office

Office européen des brevets

Publication number: **0 309 616**
A1

# EUROPEAN PATENT APPLICATION

Application number: **87118751.4**

Int. Cl.4: **A61F 9/02**

Date of filing: **17.12.87**

Priority: **02.10.87 IT 362987**

Date of publication of application:
**05.04.89 Bulletin 89/14**

Designated Contracting States:
**AT BE CH DE ES FR GB GR LI LU NL SE**

Applicant: **Grazia, Ezio**
**via della Pieve 45**
**Castenaso (Bologna)(IT)**

Inventor: **Grazia, Ezio**
**via della Pieve 45**
**Castenaso (Bologna)(IT)**

Representative: **Sassatelli, Franco T., Dr.**
**c/o INIP via Ruggi 5**
**I-40137 Bologna(IT)**

### Transparent eye screen for medical practices.

The invention foresees a transparent and flexible screen (1) which covers the upper part of the visage, with a lower void (2) for the nose, to fit in different ways on the head. The above screen (1), combined with a conventional air filter mask, forma a single use device for protecting the visage in medical practices.

EP 0 309 616 A1

FIG. 1

## Transparent eye screen for medical practices.

The invention refers to an eyes shielding screen, as well as most part of the visage. The employ of the above screen on a conventional mask with air filter enables to have a contrivance for a complete visage screen to avoid infections and, in any case, the contact with different kinds of substances during the medical and laboratory interventions and treatments. The means is for single-use employ and foreseen both for operators and patients. To protect the visage from sprinkles of substances, such as for instance blood and urine, and to avoid the contact with anatomical parts and sanitary equipments which could turn out disagreable, devices are at present used for trim maintenance and for repeated employ. These equipments show a stiff screening shape to fit on the ears with conventional spectacle sides. Such tools have two inconveniences: they do not screen from sprays and several contacts on the sides since they are held by spectable sides only and must be carefully cleaned and sterilized after the use. In such conditions, the immediate re-employ of them is not allowed. This compels the user to avail himself of many devices of this kind in order to ensure the timely possibility of employ.

The invention device avoids such inconveniences by adopting a single use screen, with limited size and weight, to easily compact in deliverers of different kinds, and to singularly manufacture at low costs. The employ of means of the tupe "use and throw away" ensures a continuity of intervention and avoids any possible restoration phases. Furthermore, since the system consists of two disjoined and light type devices, it allows the possibility of easy transport and, in case of no intervention, to free the nose and mouth, disengaging the upper binding of the mask, keeping at the same time the fitting condition of the device.

Substantially the device consists of a flexible transparent screen which covers the upper part of the visage with small side elastic cables which pass on the ears for fitting, and of a lower hollow for imposing it on the nose. This device, used in connection with a conventional mask with air filter, determinates a single use device which carries out the complete protection of the visage with stable fitting maintenance. The disposition of the lower end part of the screen on the already fitted mask, allows a screening continuity of the device as if it were a single whole. The flexible conformation of the screen, fitted not in contact with the visage in the reentering parts, enables to wear the conventional spectacles in the relevant interspace. In a version, the transparent flexible screen is carried out with a bore in either link or right ocular correspondence for fitting an endoscope. Since screen and mask are disjoined, it is possible to change their singular position on the visage, when not used, to free the relevant parts, however still maintaining the imposing condition of the device on the head.

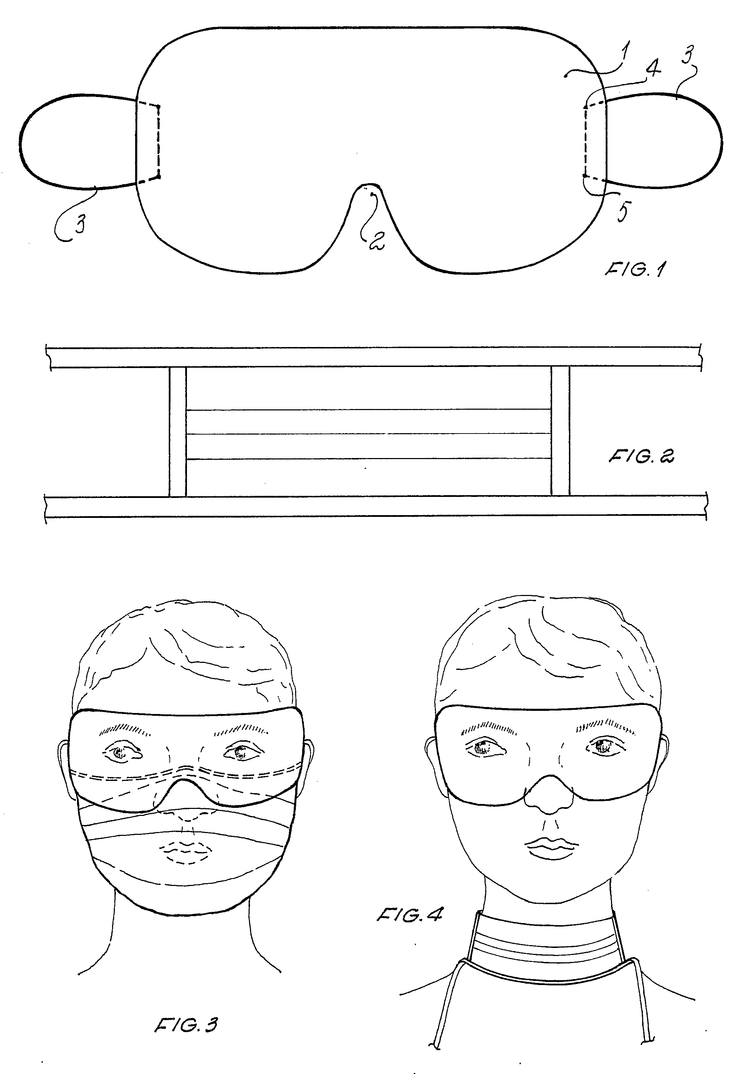

An execution form is illustrated in a merely indicative and, therefore, not limiting way, in the drawings of table 1. With reference to this table, fig. 1 is the front view of the transparent flexible screen, namely the object of the invention. Symmetrical ly fitted, the elastic cables can be noted for mounting the screen on the ears. Fig. 2 shows a conventional surgical mask which completes the protecting device along with the above mentioned screen. Fig. 3 is the front view of the device in use condition. Fig. 4 is analogous to the previous view, but with the free lower part of the visage after having untied the upper bond of the filtering mask from the head.

In the version, the transparent flexible screen uses the special shape 1 with lower hollow 2 and side elastic cables 3 passing into the bores 4 and 5. For manual fitting, the cables 3 are brought in expansion in a closed line and fitted on the ears.

The screen shape the binding means on the head, the materials, the size of the nose hollow and anything else in this connection may be foreseen in different ways.

## Claims

1) Transparent eye screen for medical practices, characterized by the fact that it consists of flexible material (1) which covers the upper part of the visage. Side elastic passing cables (3) furthermore are foreseen for fixing the screen on the ears, as well as a lower hollow (2) for imposing it on the nose. The said screen (1), used in combination over a conventional air filter mask, determines a device for single use carrying out the complete visage protection with steady fitting maintenance.

2) Transparent eye screen for medical practices, as per claim 1, characterized by the fact that the cheap costs of both the screen (1) and of the mask with which it is combined, allows the single-use employ of the assembly.

3) Transparent eye screen for medical practices, as per claim 1, characterized by the fact that the disposition of its lower end part on the already fitted mask enables a screening continuity of the device.

4) Transparent eye screen for medical practices, as per claim 1, characterized by the fact that its flexible conformation, which allows a contact with the visage in the parts to reenter, enables the employ of conventional spectacles in the relevant interspace.

5) Transparent eye screen for medical practices, as per claim 1, characterized by the fact that in a version a bore has been carried out in correspondence with either right of left eye for the employ of an endoscope.

6) Transparent eye screen for medical practices, as per claim 1, characterized by the fact that, since it is disjointed from the mask, its position on the visage may singularly change when not used.

FIG.1

FIG.2

FIG.3

FIG.4

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| Y | US-A-3 310 812 (GAISSER) * Column 1, lines 56-60; column 2, lines 54-56; column 3, lines 1-4; figures * | 1-6 | A 61 F 9/02 |
| Y | DE-A-3 030 451 (MÜLLER) * Page 3, line 26 - page 4, line 4; figures * | 1-6 | |
| Y | AU-A- 58 287 (BINI)(1973) * Page 2, lines 1-3; page 2, line 24 - page 3, line 2 * | 2 | |
| A | US-A-2 762 368 (BLOOMFIELD) | | |
| A | US-A-2 056 753 (WAGNER) | | |

TECHNICAL FIELDS
SEARCHED (Int. Cl.4)

A 61 F
A 41 D

The present search report has been drawn up for all claims

| Place of search | Date of completion of the search | Examiner |
|---|---|---|
| THE HAGUE | 31-05-1988 | GLAS J. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)